# FASCICULE DE BREVET EUROPEEN

(11) **EP 2 536 703 B1**
(45) Date de publication et mention de la délivrance du brevet: **09.04.2014**
(21) Numéro de dépôt: 11712919.7
(22) Date de dépôt: 16.02.2011
(51) Int. Cl.: C07D 311/56, A01N 49/00

(54) **Composés rodenticides, composition les comprenant et leur utilisation pour lutter contre les rongeurs nuisibles**
Rodentizide Verbindungen, Zusammensetzung davon und ihre Verwendung zur Bekämpfung von Schadnagern
Rodenticidal compounds, composition thereof and their use for controlling harmful rodents

(30) Priorité: 18.02.2010 FR 1051155
(43) Date de publication de la demande: 26.12.2012
(73) Titulaire: Liphatech, 47480 Pont-du-Casse (FR)
(72) Inventeur: LASSEUR, Romain, F-69210 Bully (FR); BENOIT, Etienne, F-69009 Lyon (FR); BERNY, Philippe, F-69670 Vaugneray (FR); BESSE, Stéphane, F-69340 Francheville (FR)
(74) Mandataire: Cabinet BARRE LAFORGUE & associés
(86) Numéro de dépôt international: PCT/FR2011/050326
(87) Numéro de publication internationale: WO 2011/101591

(56) Documents cités:
- WO-A1-99/01446
- US-A- 2 872 457
- US-A- 2 952 689
- GEBAUER ET AL: "Synthesis and structure-activity relationships of novel warfarin derivatives", BIOORGANIC & MEDICINAL CHEMISTRY, PERGAMON, GB LNKD- DOI:10.1016/J.BMC.2007.01.014, vol. 15, no. 6, 15 février 2007 (2007-02-15), pages 2414-2420, XP005890907, ISSN: 0968-0896

## Description

La présente invention concerne le domaine des produits rodenticides c'est-à-dire des produits qui ont la propriété de tuer certains rongeurs.

L'invention vise en particulier de nouvelles molécules présentant un effet rodenticide ainsi que les compositions les intégrant. Elle se rapporte également à l'utilisation de ces molécules et compositions pour lutter contre des rongeurs nuisibles.

Les dégâts causés par certains nuisibles comme les rats ou les souris sont connus depuis longtemps, et les risques liés à leur présence sont graves.

Outre les dégâts matériaux qu'ils peuvent causer en particulier sur les installations électriques, les rongeurs nuisibles constituent surtout un fléau sanitaire important. Directement ou par leurs parasites, ils sont à l'origine de nombreuses maladies transmises à l'homme et aux animaux domestiques, comme le tétanos, la streptobacillose, la pasteurellose, la leptospirose, la salmonellose, des fièvres hémorragiques, ou encore la peste.

C'est pourquoi, depuis de nombreuses années, différents systèmes de désinfestation ont été développés : des systèmes physiques par exemple à base de trappes mécaniques, de colles ou encore d'ultrasons, et des systèmes chimiques, notamment des produits répulsifs, chimiostérilisants ou rodenticides. Même si l'utilisation de systèmes physiques se développe, le contrôle de populations de rongeurs est toujours majoritairement réalisé par le biais de méthodes de lutte utilisant des rodenticides, notamment pour des raisons d'efficacité en cas d'infestations massives.

Les rodenticides appartiennent à des familles chimiques très diverses : anticoagulants, convulsivants, cardiotoxiques, cytotoxiques ou hypercalcémiants. La plupart des rodenticides utilisés aujourd'hui sont des anticoagulants, en particulier des anticoagulants dérivés de l'hydroxy-4-coumarine ou coumaphène comme le coumachlore, le coumatétralyl, le difenacoum ou le brodifacoum, la diféthialone ou la bromadiolone et des anticoagulants dérivés de l'indane-dione-1,3 comme la chlorophacinone, la diphacinone.

Gebauer et al, (2007), « Bioorganic & Medicinal Chemistry », 15(6), 2414-2420, WO 99/01446, US 2872457 et US 2 952 689 décrivent des composes dérivés de la 4-hydroxy-1-benzo(thio)pyran-2-one.

Le métabolisme des anticoagulants se caractérise par une bonne absorption orale et une distribution essentiellement hépatique. Ces produits sont donc très efficaces. Ils agissent par accumulation pendant plusieurs jours jusqu'à créer la mort de l'animal par anémie aiguë, sans souffrance et sans éveiller la méfiance de ses congénères envers le produit.

Toutefois, les anticoagulants rodenticides existants actuellement ont une rémanence élevée et persistent longtemps dans les cadavres des rongeurs qui les ont ingérés. Or, comme ce sont des produits toxiques non sélectifs, les espèces prédatrices des rongeurs, en particulier rapaces et petits mammifères sauvages ou domestiques, peuvent s'intoxiquer elles-mêmes avec ces anticoagulants stockés dans les cadavres.

Il subsiste donc un besoin pour un produit qui permette de lutter efficacement contre les rongeurs tout en préservant la faune non cible des intoxications secondaires.

Pour y répondre la présente invention propose un nouveau composé qui présente une action rodenticide au moins aussi efficace que les anticoagulants existants, mais dont la durée de stockage dans le foie n'est pas suffisante pour entraîner des intoxications secondaires.

En particulier, l'invention vise un composé répondant à la formule (I) : 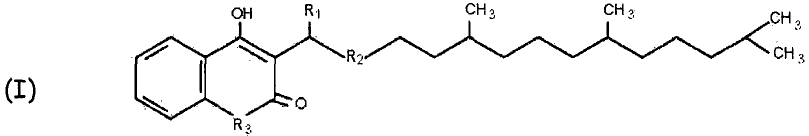 ainsi que ses isomères, en particulier énantiomères, diastéréoisomères, tautomères ou mélanges d'isomères en toutes proportions, dans laquelle :
- R1 représente : H ou 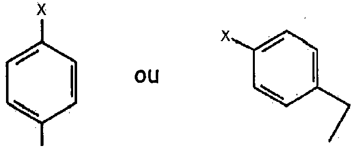
- R2 représente: 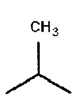 ou 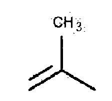 ou 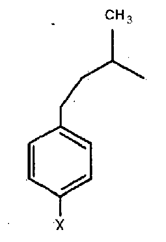
- R3 représente : O ou S ou 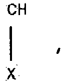
avec X = H, OH, Cl, Br, F ou NO₂,
X pouvant être différent ou identique dans R1, R2 ou R3

L'invention vise aussi un procédé de production d'un composé de formule (I) à partir de 4-hydroxy-coumarine.

Un autre objet de l'invention concerne des compositions rodenticides comprenant au moins un composé répondant à la formule (I). Préférentiellement ces compositions se présentent sous forme de poudres, de blocs, de gels, de pâtes, de céréales imprégnées, ou encore sous forme liquide.

Enfin l'invention vise également l'utilisation d'un composé de formule (I) ou d'une composition l'incluant pour le contrôle de populations de rongeurs. Avantageusement, les molécules selon l'invention allient une réelle efficacité rodenticide sur tous les rongeurs et une très faible rémanence hépatique. Leur utilisation permet donc une lutte efficace contre les rongeurs tout en réduisant considérablement le risque d'intoxications secondaires.

D'autres caractéristiques et avantages ressortiront de la description en détail qui va suivre de l'invention.

Selon un premier aspect, l'invention se rapporte donc à un composé selon la revendication 1.

Les composés répondant à cette formule (I) et ses isomères présentent une activité rodenticide.

### Préférentiellement R3 représente un oxygène O.

Selon une variante préférée et particulièrement adaptée, l'invention vise un composé spécifique répondant à la formule (I) dans laquelle :
- R1 représente H,
- R3 représente O, et
- R2 représente :

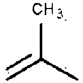

Ce composé spécifique présente la formule (II) suivante :

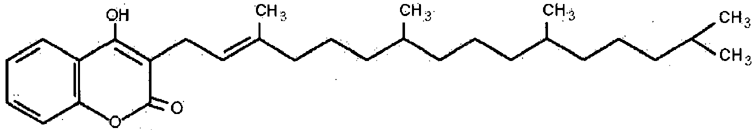

Selon une autre variante, le composé selon l'invention répond à la formule (I) dans laquelle :
- R1 représente 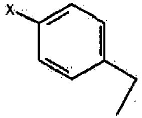
- R2 représente 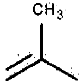
- R3 représente O.

Ce composé spécifique présente la formule (III) suivante :

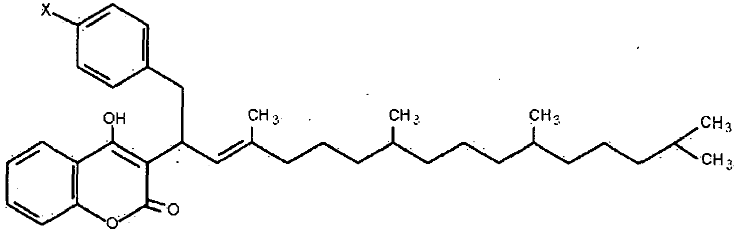

Le composé selon l'invention peut également répondre à la formule (I) dans laquelle :
- R1 représente 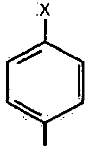
- R2 représente 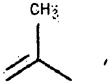 et
- R3 représente O.

Ce composé spécifique présente la formule (IV) suivante :

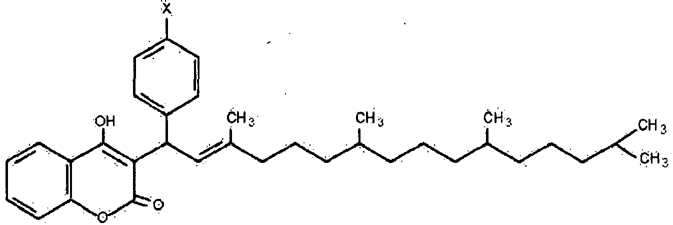

Selon un autre mode de réalisation, le composé selon l'invention peut également répondre à la formule (I) dans laquelle :
- R1 représente H,
- R3 représente O, et
- R2 représente

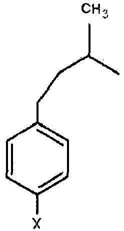

Ce composé particulier présente la formule (V) suivante :

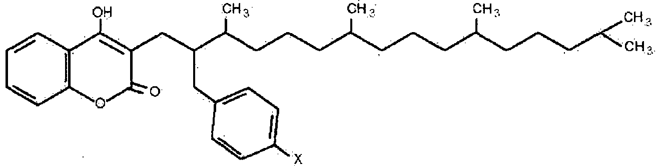

Les composés selon l'invention peuvent être obtenus en greffant une chaîne aliphatique latérale ramifiée et potentiellement plus ou moins substituée, sur un 4 hydroxycoumarine.

Le procédé d'obtention d'un composé de formule (I) selon l'invention peut donc être schématisé comme suit :

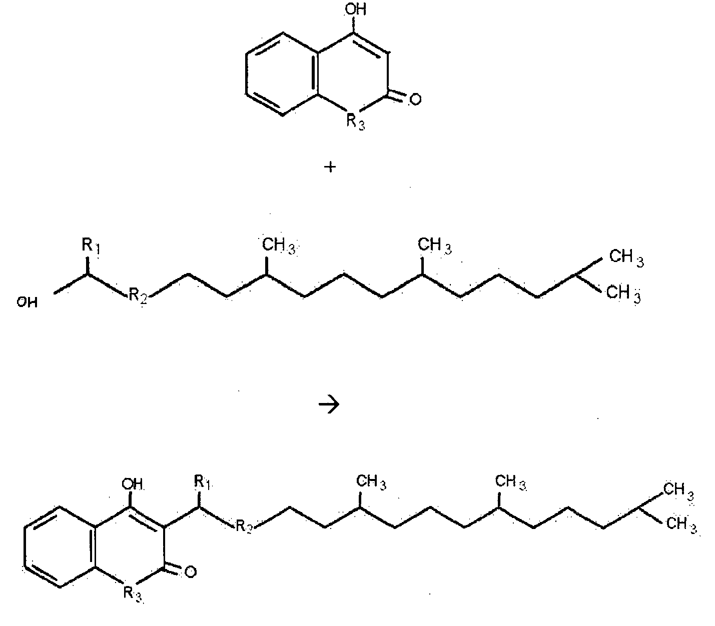

Avec :
- R1 représentant : H ou 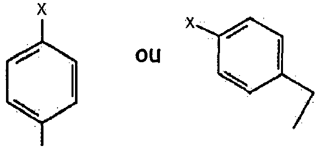
- R2 représentant : 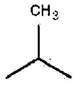 ou 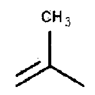 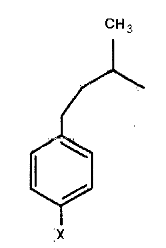
- R3 représentant : 0 ou S ou

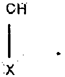

X représentant H, OH, Cl, Br, F ou NO₂, X pouvant être différent ou identique dans R1, R2 ou R3.

Le greffage de la chaîne aliphatique peut être réalisé par tout procédé de greffage adapté. Il peut en particulier être réalisé par une réaction d'addition de Michael à l'aide d'un dérivé bromé.

A titre d'exemple, le composé particulier de formule (II) peut être obtenu par la mise en oeuvre des étapes suivantes :
- synthèse de bromure de phytyl à partir de phytol et de bromure de carbone, et
- synthèse du 3-phytyl-4-hydroxy-coumarine à partir de 4-hydroxy-coumarine et de bromure de phytyl en milieu alcalin éthanolique.

La synthèse du bromure de phytyl à partir de phytol et de bromure de carbone, est une réaction d'halogénation sur un alcool primaire, qui se déroule à température ambiante en présence de triphényl phosphine et de tétrabromure de carbone en milieu dichlorométhane pendant deux heures. Le bromure de phytyl synthétisé est purifié par des lavages successifs avec des solutions de NaHCO₃, H₂O et de saumure. Le produit brut obtenu est concentré et utilisé tel quel.

La synthèse du 3-phytyl-4-hydroxy-coumarine à partir de 4-hydroxy-coumarine et de bromure de phytyl est une réaction de C-alkylation qui correspond à l'attaque de l'halogénure d'alkyl sur un carbone chargé du cycle coumarinique en position 3. La réaction a lieu en milieu alcalin de façon à permettre dans un premier temps la formation d'un ion phénolate qui contribue à délocaliser fortement la charge électronique portée par le carbone en position 3. Dans un second temps, le bromure de phytyl réagit en milieu éthanolique. Le milieu réactionnel est ensuite purifié par succession d'extractions liquide-liquide sélectives. On obtient alors deux isomères (cis et trans) du composé de formule (II).

Les composés de formule (I) selon l'invention, en particulier les composés spécifiques de formule (II), (III), (IV) ou (V), ainsi que leurs isomères, présentent un effet rodenticide important, même à faible concentration.

Ils peuvent être incorporés dans des compositions destinées à lutter contre les rongeurs nuisibles.

L'invention a donc également pour objet des compositions rodenticides incluant comme matière active au moins un composé de formule (I).

Préférentiellement l'invention vise des compositions rodenticides comprenant entre 0,001 et 50% en poids d'un composé répondant à la formule (I), encore plus préférentiellement entre 0,001 et 20%.

Les compositions selon l'invention peuvent se présenter sous toute forme adaptée à la lutte contre les rongeurs nuisibles. Elles peuvent notamment se présenter sous forme de concentrat liquide, de poudre, de céréales, de gels, de pâtes, ou d'appâts extrudés comme des pellets ou des blocs.

En plus du composé de formule (I), les compositions selon l'invention contiennent plusieurs excipients : un ou plusieurs excipients composant la matrice contenant le composé rodenticide, et un ou plusieurs excipients dont le rôle est technologique ou biologique.

Les excipients utilisés dans la matrice peuvent être notamment choisis parmi des huiles minérales, des glucides comme les dextrines, des matières minérales comme l'argile, des glycols et autres solvants organiques comme le DMSO, de l'eau, des matières premières céréalières comme l'orge, le blé, le maïs ou le riz, des liants comme la paraffine.

Les autres excipients souvent utilisés sont par exemple des additifs d'appétence et d'attractivité des rongeurs comme du sucre ou des arômes, des conservateurs, ou encore des amérisants destinés à réduire le risque de consommation accidentelle par les enfants ou les animaux domestiques.

Les compositions selon l'invention peuvent être fabriquées par mélange intime et homogène du ou des composés actifs de formule (I) aux ingrédients du support. Cette simplicité de réalisation est notamment possible du fait de la faible quantité de composé actif requise pour obtenir l'effet rodenticide désiré.

Le mélange de la matière active et des excipients peut être réalisé selon tous procédés adaptés, notamment par convection, diffusion et/ou cisaillement. Le mélange peut être réalisé par exemple dans des cuves avec double enveloppe permettant un refroidissement ou un chauffage du mélange le cas échéant.

Un exemple de composition adaptée selon l'invention destinée à se présenter sous forme d'un appât extrudé comprend les composés suivants :
- des matières premières céréalières (blé, orge, maïs, riz), préférentiellement entre 40 et 70% en poids de la composition,
- un liant de type paraffine ou de l'eau, préférentiellement entre 15 et 40% en poids de la composition,
- un composé de formule (I), préférentiellement entre 0,001 et 50% en poids de la composition,
- un amérisant du type benzoate de dénatorium,
- un ou plusieurs conservateurs de la famille des acides organiques et de leurs sels comme l'acide ascorbique, l'acide propionique ou le sorbate de potassium, et
- un colorant, préférentiellement le colorant vert PG 7.

La fabrication du support extrudé en procédé continu comprend les étapes suivantes :
- mélange des ingrédients à l'aide d'un mélangeur par convection,
- extrusion à l'aide d'un extrudeur monovis ou extrudeur bivis, de diamètre 30mm à 100mm, entre 55°C et 95°C,
- formage,
- découpe d'appâts de masse comprise entre 5 et 100g,
- stabilisation par traitement thermique : refroidissement par air pulsé pendant 30 minutes ou séchage à convection pendant 2 à 10 heures, et
- conditionnement.

Les composés de formule (I) et les compositions selon l'invention peuvent être utilisés pour lutter contre les rongeurs nuisibles.

Les compositions rodenticides comprenant les composés de formule (I) peuvent être appliquées en prévention ou en traitement dans des zones à protéger d'attaques de rongeurs. Elles peuvent éventuellement être insérées ou fixées dans des boîtes d'appâtage adaptées à la lutte des rongeurs afin de sécuriser leur emploi.

Avantageusement, les composés de formule (I) selon l'invention présentent une très bonne efficacité rodenticide, au moins aussi importante que les rodenticides anticoagulants existants, tout en présentant une très faible rémanence hépatique. En effet, les composés selon l'invention se stockent dans le foie pendant un temps très limité.

Leur utilisation permet donc une lutte efficace contre les rongeurs tout en préservant la faune non cible des intoxications secondaires.

Ils peuvent être utilisés notamment dans l'environnement domestique, en milieu rural, professionnel, urbain, dans les lieux de restauration ou d'accueil du public ou encore dans l'industrie agroalimentaire pour lutter contre les rats, souris, mulots, surmulots et/ou tout autre rongeur nuisible.

La dose de composé à administrer dépend du rongeur visé. Elle est préférentiellement comprise entre 0,5mg et 100mg en fonction du rongeur concerné.

L'invention est à présent illustrée par un exemple et des résultats d'essais montrant les effets revendiqués.

### EXEMPLE DE PROCEDE D'OBTENTION D'UN COMPOSE DE FORMULE (I) SPECIFIQUE, LE COMPOSE DE FORMULE (II)

Le procédé consiste en la mise en oeuvre de deux étapes : synthèse de bromure de phytyl à partir de phytol et de tétrabromure de carbone, et synthèse du composé de formule (II), le 3-phytyl-4-hydroxy-coumarine, à partir de 4-hydroxy-coumarine et de bromure de phytyl en milieu alcalin éthanolique.

### a. Synthèse du BROMURE de PHYTYL

Le bromure de phytyl peut être obtenu par la mise en oeuvre des étapes suivantes :
- dans un ballon à col rodé introduire : du phytol (10mM) et du dichlorométhane contenant de la triphényl phosphine ; un excès de 20 à 30 % est nécessaire pour consommer entièrement le phytol,
- mettre sous agitation magnétique,
- ajouter en une seule fois du tétrabromure de carbone, la solution obtenue est limpide et légèrement jaune claire,
- laisser réagir pendant 2 heures à température ambiante.

La purification du milieu réactionnel est ensuite réalisée par extractions liquide-liquide :
- ajouter 50 ml d'une solution saturée en NaHCO₃,
- agiter et éliminer la phase aqueuse,
- laver la phase organique avec de l'eau distillée,
- agiter et éliminer les phases aqueuses,
- évaporer à sec sous courant d'azote la phase organique,
- reprendre le résidu sec avec de l'hexane ou du cyclo-hexane,
- filtrer la phase hexanique sur filtre Whatman,
- laver le précipité avec de l'hexane ou du cyclo-hexane, puis
- évaporer à sec l'hexane ou le cyclo-hexane.

Un résidu huileux orangé est obtenu.

### b. Synthèse du 3-PHYTYL-4-OH-COUMARINE

Le 3-phytyl-4-hydroxy-coumarine peut être obtenu par la mise en oeuvre des étapes suivantes :
- dans un ballon introduire : de la 4 hydroxy coumarine (10 mM), 4mM d'une solution de NaOH 1M (4 mM), et 10mM d'une solution de NaOH 10M pour une solubilisation totale, de façon à obtenir une solution légèrement jaune claire,
- mettre sous agitation magnétique à 70°C pendant 30 minutes, pour permettre la formation du phénolate de coumarine,
- ajouter du bromure de phytyl (7,3 mM), puis de l'éthanol,
- mettre à 70°C pendant 2 heures sous agitation magnétique, le milieu réactionnel vire progressivement vers le rouge foncé.

La purification du milieu réactionnel est réalisée par extractions liquide-liquide (lors de ces opérations toutes les phases liquides sont chromatographiées en HPLC) :
- ajouter de la soude 1 M dans le milieu jusqu'à obtention d'un milieu à pH 14,
- laver avec du toluène, laisser décanter et récupérer l'interface huileuse avec la phase alcaline,
- extraire les phases toluèniques avec une solution de NaOH 1M, et les éliminer,
- rassembler les phases alcalines de couleur rouge et extraire à l'aide d'acétate d'éthyle,
- éliminer la phase aqueuse alcaline, et laver la phase acétate d'éthyle avec une solution de HCl,
- éliminer les phases aqueuses acides et laver la phase acétate d'éthyle avec de l'eau distillée,
- éliminer les phases aqueuses et filtrer l'acétate d'éthyle sur sulfate de sodium anhydre,
- laver le sulfate de sodium avec de l'acétate d'éthyle, et
- évaporer à sub-sac sous courant d'azote l'acétate d'éthyle.

Le produit final se présente sous forme d'une huile jaune orangé. Les molécules obtenues répondent à la formule (II).

### EVALUATION DE L'EFFICACITE ET DE LA REMANENCE SUR DES RATS DU PRODUIT DE FORMULE (II)

L'efficacité rodenticide et la rémanence des composés selon l'invention ont été testées en comparaison avec le produit rodenticide de référence Bromadiolone, la Bromadiolone étant le moins rémanent de tous les anti-coagulants de deuxième génération actuellement utilisés dans la lutte contre les rongeurs.

Pour cet essai, le composé de formule (II) obtenu par la mise en oeuvre du procédé exposé précédemment est utilisé.

Le protocole opératoire est exposé en suivant. 3 lots de 5 rats ont été utilisés pour le composé de formule (II) et 2 lots de 4 rats pour le Bromadiolone.

### Pour le composé de formule (II) :

3 doses de 10mg/kg de rat ont été administrées en intra péritonéal successivement à T0, T24h et T48h.
1 lot de rats a été utilisé pour suivre la mortalité occasionnée par les 3 administrations du composé de formule (II) selon l'invention.
1 lot de rats a été utilisé pour suivre le temps de coagulation entre 24h et 120h après la première injection. En effet, le temps de coagulation est le premier paramètre qui varie et tend vers l'infini sous l'action d'un anticoagulant.
1 lot de rats a été utilisé pour réaliser un dosage de résidus du composé de formule (II) dans le foie à T72h, T96h et T120h.

### Pour le Bromadiolone :

Des doses de 2 mg/kg/j ont été administrées pendant 4 jours.
1 lot de rats a été utilisé pour déterminer la mortalité occasionnée à T120h.
Un dosage de résidus de Bromadiolone dans le foie à T120h a été réalisé.
1 lot de rats a été utilisé pour suivre le temps de coagulation entre 24h et 120h après la première injection.

### Résultats

Les résultats obtenus sont présentés ci-dessous :

| | | T0 | T24h | T48h | T72h | T96h | T120h | Total Mortalité |
|---|---|---|---|---|---|---|---|---|
| | Suivi mortalité | / | 2 morts | 2 morts | 1 mort | / | / | 100% |
| Composé selon l'invention | Suivi temps coagulation (secondes) | Infini | Infini | Infini | Infini | Infini | 120 | / |
| | Suivi résidus foie (µg/g) | / | / | / | 0,65 | 0,51 | 0,37 | / |
| Bromadiolone | Suivi Mortalité | / | / | / | / | 2 morts | 2 morts | 100% |
| | Suivi temps coagulation (s) | Infini | Infini | Infini | Infini | Infini | Infini | / |
| | Suivi résidus foie (µg/g) | / | / | / | / | / | 4,2 | / |

Ces résultats montrent tout d'abord que l'injection de composés selon l'invention permet d'occasionner la mort de 100% des rats traités, tout comme le produit rodenticide de référence.

Par ailleurs, 120 heures après la première injection, le temps de coagulation reste infini pour le Bromadiolone alors qu'il décroît à 120 secondes pour le composé selon l'invention. Cela traduit une métabolisation rapide du composé de formule (II), phénomène que l'on n'observe pas pour la Bromadiolone.

De plus, on constate une décroissance rapide du dosage de résidus des composés selon l'invention entre 72h et 120h après la première injection, et une faible rémanence dans le foie des rats. En comparaison, les résultats obtenus avec le Bromadiolone 120 heures après la première injection montrent la présence de 11 fois plus de résidus, alors que 30mg de composé de formule (II) ont été administrés contre seulement 8mg de Bromadiolone.

Ces résultats mettent donc en évidence un métabolisme rapide des composés selon l'invention chez les rongeurs, en parallèle d'une haute efficacité sur cette cible.

## Revendications

1. Composé répondant à la formule (I) : 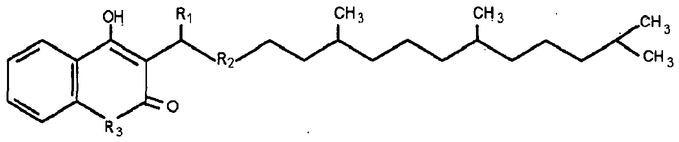 ainsi que ses énantiomères, diastéréoisomères, tautomères ou mélanges de ces derniers en toutes proportions,
dans laquelle :
- R1 représente : H ou 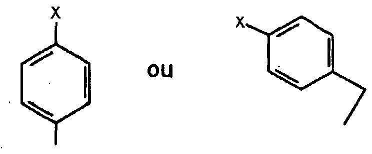
avec X = H, OH, Cl, Br, F ou NO_{2,}
- R2 représente : 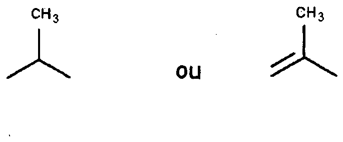 ou 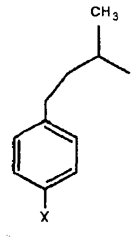
avec X = H, OH, CI, Br, F ou NO₂,
- R3 représente : O ou S ou 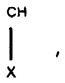 avec X = H, OH, Cl, Br, F ou NO₂.

2. Composé selon la revendication 1, **caractérisé en ce qu'**il répond à la formule (II) suivante :

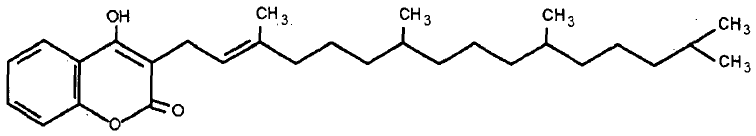

3. Composé selon la revendication 1, **caractérisé en ce qu'**il répond à la formule (III) suivante :

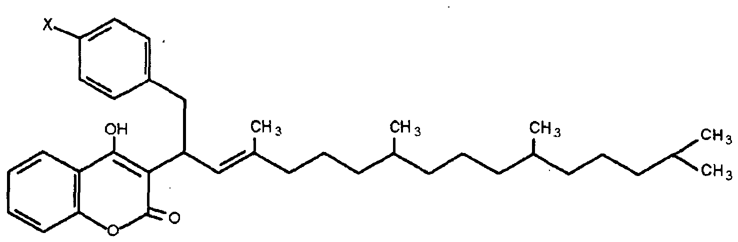

4. Composé selon la revendication 1, **caractérisé en ce qu'**il répond à la formule (IV) suivante :

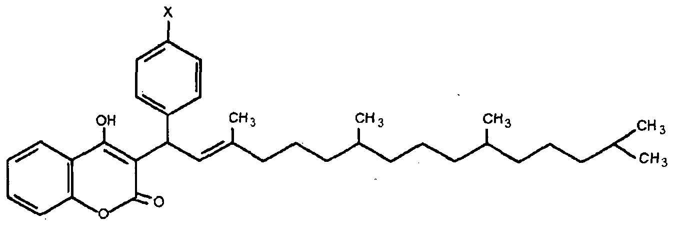

5. Composé selon la revendication 1, **caractérisé en ce qu'**il répond à la formule (V) suivante :

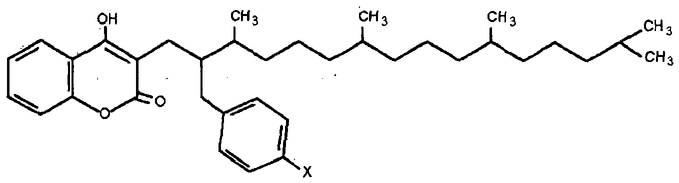

6. Composition rodenticide, **caractérisée en ce qu'**elle comprend au moins un composé selon l'une des revendications 1 à 5.

7. Composition rodenticide selon la revendication 6, **caractérisée en ce qu'**elle comprend entre 0,001 et 50% en poids d'un composé répondant selon l'une des revendications 1 à 5.

8. Composition rodenticide selon la revendication 6 ou 7, **caractérisée en ce qu'**elle se présente sous forme de concentrat liquide, de poudre, de céréales, de gels, de pâtes, ou d'appâts extrudés comme des pellets ou des blocs.

9. Utilisation d'un composé selon l'une des revendications 1 à 5, ou d'une composition rodenticide selon l'une des revendications 6 à 8, pour lutter contre les rongeurs nuisibles.

10. Utilisation d'un composé selon l'une des revendications 1 à 5, ou d'une composition rodenticide selon l'une des revendications 6 à 8, dans l'environnement domestique, en milieu rural, professionnel, urbain, dans les lieux de restauration ou d'accueil du public ou encore dans l'industrie agroalimentaire pour lutter contre les rats, souris, mulots, surmulots et/ou tout autre rongeur nuisible.

## Patentansprüche

1. Verbindung, entsprechend der folgenden Formel (I) : 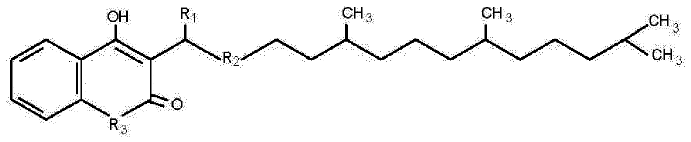 ebenso wie ihre Enantiomere, Diastereoisomere, Tautomere oder Mischungen dieser Letzteren in allen Proportionen.
wobei:
- R1 Folgendes darstellt: H oder 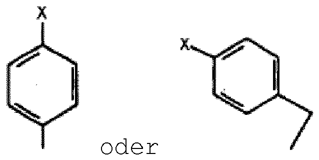
wobei X = H, OH, Cl, Br, F oder NO₂,
- R2 Folgendes darstellt: 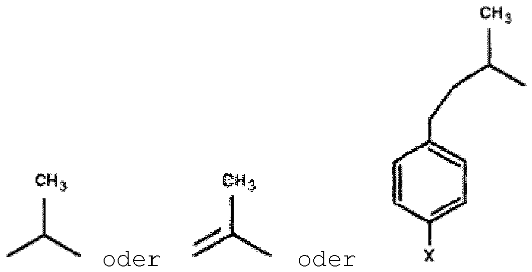
wobei X = H, OH, Cl, Br, F oder NO₂,
- R3 Folgendes darstellt: O oder S oder 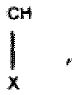 wobei X = H, OH, Cl, Br, F oder NO₂,

2. Verbindung nach Anspruch 1, **dadurch gekennzeichnet, dass** sie der folgenden Formel (II) entspricht:

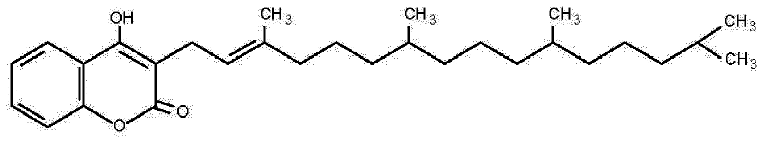

3. Verbindung nach Anspruch 1, **dadurch gekennzeichnet, dass** sie der folgenden Formel (III) entspricht:

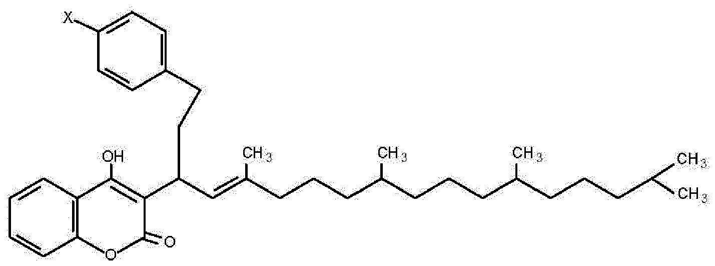

4. Verbindung nach Anspruch 1, **dadurch gekennzeichnet, dass** sie der folgenden Formel (IV) entspricht:

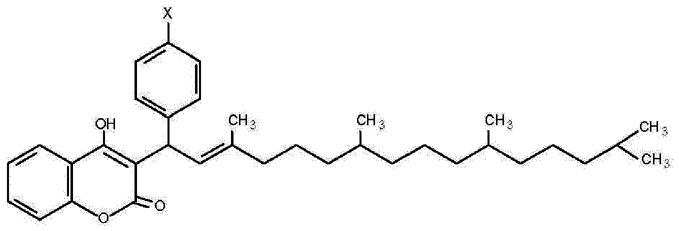

5. Verbindung nach Anspruch 1, **dadurch gekennzeichnet, dass** sie der folgenden Formel (V) entspricht:

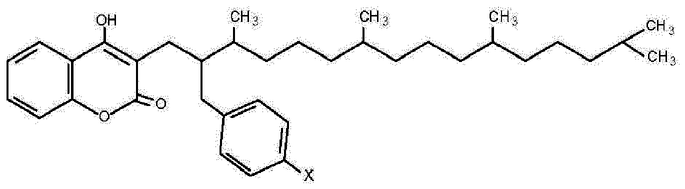

6. Rodentizide Zusammensetzung, **dadurch gekennzeichnet, dass** sie mindestens eine Verbindung nach einem der Ansprüche 1 bis 5 umfasst.

7. Rodentizide Zusammensetzung nach Anspruch 6, **dadurch gekennzeichnet, dass** sie zwischen 0,001 und 50 Gew% einer Verbindung nach einem der Ansprüche 1 bis 5 umfasst.

8. Rodentizide Zusammensetzung nach Anspruch 6 oder 7, **dadurch gekennzeichnet, dass** sie die Form eines flüssigen Konzentrats, eines Pulvers, von Getreide, von Gels, von Pasten oder extrudierten Ködern wie z.B. Pellets oder Blöcken aufweist.

9. Verwendung einer Verbindung nach einem der Ansprüche 1 bis 5 oder einer rodentiziden Zusammensetzung nach einem der Ansprüche 6 bis 8, um gegen schädliche Nager zu kämpfen.

10. Verwendung einer Verbindung nach einem der Ansprüche 1 bis 5 oder einer rodentiziden Zusammensetzung nach einem der Ansprüche 6 bis 8 in der häuslichen Umgebung, im ländlichen, professionellen, städtischen Umfeld, in Gastronomielokalen oder Lokalen zum Empfang von Publikum oder auch in der Agrar- und Ernährungsindustrie, um gegen Ratten, Mäuse, Waldmäuse, Wanderratten und/oder alle anderen schädlichen Nager zu kämpfen.

## Claims

1. Compound corresponding to the formula (I): 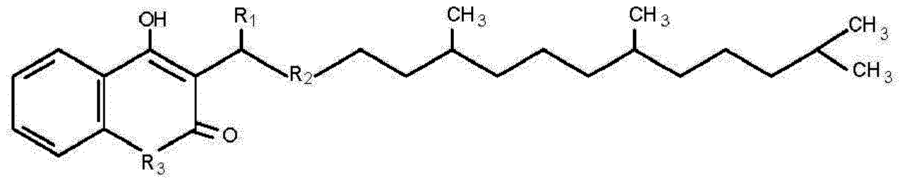 and enantiomers, diastereoisomers, tautomers or mixtures of isomers in all proportions,
in which:
- R1 represents: H or 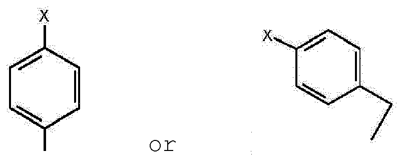
where X = H, OH, Cl, Br, F or NO₂,
- R2 represents: 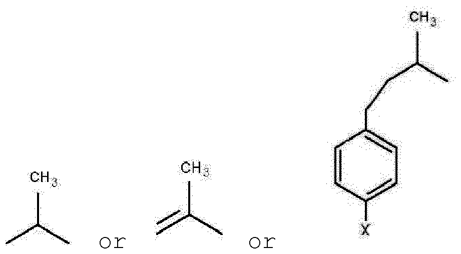
where X = H, OH, Cl, Br, F or NO₂,
- R3 represents: O or S or 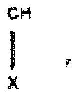 where X = H, OH, Cl, Br, F or NO₂

2. Compound according to claim 1, **characterized in that** it corresponds to the following formula (II):

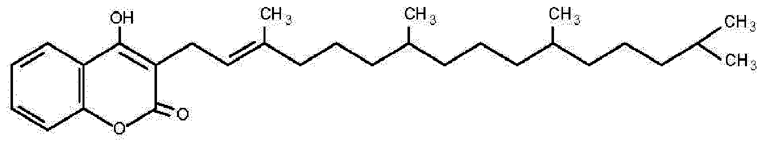

3. Compound according to claim 1, **characterized in that** it corresponds to the following formula (III):

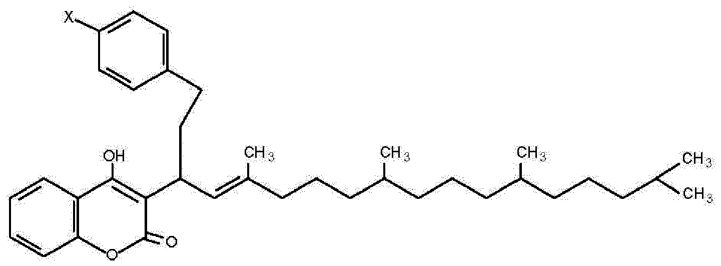

4. Compound according to claim 1, **characterized in that** it corresponds to the following formula (IV):

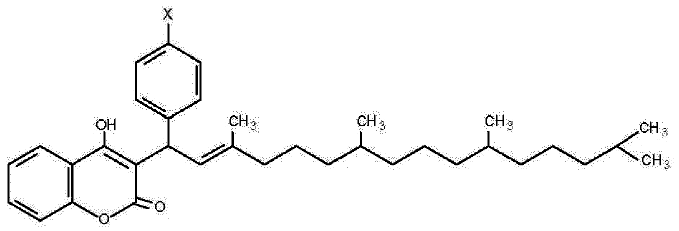

5. Compound according to claim 1, **characterized in that** it corresponds to the following formula (V):

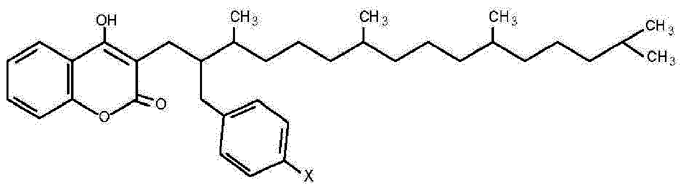

6. Rodenticidal composition, **characterized in that** it comprises at least one compound according to one of claims 1 to 5.

7. Rodenticidal composition according to claim 6, **characterized in that** it comprises between 0.001 and 50 % by weight of a corresponding compound according to one of claims 1 to 5.

8. Rodenticidal composition according to claim 6 or 7, **characterized in that** it is in the form of a liquid concentrate, powder, grains, gels, pastes, or extruded baits, such as pellets or blocks.

9. Use of a compound according to one of claims 1 to 5 or of a rodenticidal composition according to one of claims 6 to 8 for combating rodent pests.

10. Use of a compound according to one of claims 1 to 5 or of a composition according to one of claims 6 to 8 in the domestic environment, in a rural, professional, urban medium, in public catering or reception places or also in the food processing industry, for combating rats, mice, field mice, brown rats and/or any other rodent pest.
